(19) **Europäisches Patentamt / European Patent Office / Office européen des brevets**

(11) **EP 3 269 303 A1**

(12) # EUROPEAN PATENT APPLICATION
published in accordance with Art. 153(4) EPC

(43) Date of publication:
**17.01.2018 Bulletin 2018/03**

(51) Int Cl.:
**A61B 5/11** *(2006.01)*

(21) Application number: **16765332.8**

(22) Date of filing: **24.02.2016**

(86) International application number:
**PCT/RU2016/000098**

(87) International publication number:
**WO 2016/148601 (22.09.2016 Gazette 2016/38)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **13.03.2015 RU 2015108821**

(71) Applicant: **Healbe Corporation**
**Redwood City, CA 94063 (US)**

(72) Inventors:
- **RUBIN, Mikhail Semenovich**
  **St. Petersburg 191119 (RU)**
- **SYSOEV, Sergei Sergeevich**
  **St. Petersburg 197341 (RU)**

(74) Representative: **Reichert & Lindner**
**Partnerschaft Patentanwälte**
**Bismarckplatz 8**
**93047 Regensburg (DE)**

(54) **METHOD FOR DETERMINING THE TYPE OF MOTION ACTIVITY OF A PERSON AND DEVICE FOR IMPLEMENTING SAME**

(57) The group of inventions relates to the field of measurements aimed at studying or analyzing movements of a human body or parts thereof for diagnostic purposes, in particular the determining of types of human motor activity. To implement the method, signals generated by a three-component accelerometer attached to human body are recorded and lay the basis for calculating acceleration vector magnitudes, forming a time array of acceleration vector magnitudes, and identifying its extrema. Thereafter, duration of separate motor acts and the differences between adjacent extrema of acceleration vector magnitude for each motor act are determined by counting, consecutively from one extremum to another, the number of extrema equal to a reference number of extrema in at least one pattern, which is pre-formed for a certain type of motor activity and characterized by a specific reference number of acceleration vector magnitude extrema, a specific reference value of motor act duration, and specific reference values of difference between adjacent extrema of acceleration vector magnitude; Based on comparison results, a decision is made on whether a motor act of a certain type of motor activity is performed. The technical result pursued by this method is to make the identification of types of human motor activity more reliable.

Fig. 3

**Description**

**FIELD OF INVENTION**

The invention relates to the field of measurements aimed at studying or analyzing movements of a human body or parts thereof for diagnostic purposes, in particular for determining of types of a human motor activity.

**BACKGROUND OF THE INVENTION**

The determination of the type of human motor activity, or recognition/identification of whether a human is standing, walking, running, swimming or moves otherwise at a given instant is important for obtaining diagnostic information, in particular for assessing human health, evaluating physical exertion and activity-related energy expenditure.
Various methods for determining types of the human motor activity through the use of accelerometers attached to human body are known.
For example, application JP 2013143996 (published 25/07/2013), describes a method for determining types of human motor activity based on recording of signals from a three-component accelerometer attached to the wrist, detecting recorded signal peaks associated with contacts of the feet and the ground and motion of user's arms, and evaluating amplitudes of said peaks. The type of motor activity, such as running or walking, is determined by solving corresponding equations and comparing the results of the calculations with a predetermined threshold value. This method cannot provide a reliable identification of motor activity type, since it leaves out of consideration motion habits, which are individual for each human.
Application US 20130245470 (published 19/09/2013) describes a method for determining types of human motor activity by means of a three-component accelerometer attached to human breast. Here, the identification of motor activity type is based on the fact that at some moments of running when the runner's feet have no contact with the ground, the acceleration vector recorded by the accelerometer is directed opposite to gravity vector. At those instants, the derivative of acceleration vector magnitude has an opposite sign compared to the case when at least one foot contacts the ground. As a result, the type of movement is determined based on the derivative of acceleration vector magnitude. Said method cannot provide a reliable identification of motor activity type, since it leaves out of consideration the individual motion habits of a particular person, moreover, it is only able to distinguish running from walking.
The method closest to the claimed invention is described in application JP 2012065749 (published on 05/04/2012). Said method provides the recording of signals generated by a three-component accelerometer, which is attached to a human body and calculating the acceleration vector magnitude based thereon. Thereafter, the difference between extrema of acceleration vector magnitude is calculated and the obtained results are used to decide what type of human motor activity was performed. However, this method, like those described above, cannot provide a reliable identification of human motor activity type, since it leaves out of consideration the individual motion habits of a particular person even when he/she performs similar movements.

**SUMMARY OF THE INVENTION**

The technical object that the claimed invention is aimed to, is to develop a more reliable method and device for identifying different types of motor activity. Said object being achieved by compiling a set of reference values of amplitude and time parameters of acceleration magnitude for a particular person performing various types of motor activity, hereinafter referred to as patterns, and use them for implementing the method.
The following terms are used to describe the invention.
Human motor activity is a set of motor acts or body movements; it is the main function of human muscular system.
Types of human motor activity include walking, running, squatting, arm swinging, foot swinging, swimming, etc., performed by a person.
A motor act means a repeated movement, such as a separate step in the process of walking or running, a separate squat or arm swing, etc.
A three-component accelerometer is a technical device, which, when attached to a moving object, can generate three electric signals corresponding to the projections of moving object acceleration vector along three orthogonal axes in three-dimensional space.
Acceleration vector magnitude is an absolute value of acceleration recorded by a three-component accelerometer in the form of three signals corresponding to acceleration projections of a moving object along three orthogonal axes in three-dimensional space.
Time array of acceleration vector magnitudes is an array of values of acceleration vector magnitude for a certain (specified) time interval.
Time limits or motor act duration is a time interval from the beginning to the end of a separate motor act; or a time interval between the same phases of periodically repeated motor acts.

An acceleration vector magnitude extremum is a local extremum of acceleration vector magnitude in the time array of its values.

Number of acceleration vector magnitude extrema is the number of local extrema within the time limits or duration of an individual motor act.

A pattern is a set of parameters characterizing a type of human motor activity as a set of motor acts of a certain type, including:

a reference value of the number of extrema per motor act (for example, a step during walking or running, a swing of an arm, etc.);

a reference value of motor act duration, which is represented by minimum and maximum values;

a reference value of difference between adjacent extrema, which is represented by minimum and maximum values of each extremum with respect to the next extremum per one motor act.

A subset of patterns is an array of patterns of the same motor activity type that differ in their reference number of extrema per motor act.

Said sets of parameters (patterns) are formed in advance when customized for various types of motor activity, which can be identified using the method proposed in the claimed invention.

One of the targets of said invention is to develop a method for identifying types of human motor activity, wherein:

signals from a three-component accelerometer, which is attached to the human body and is capable of generating signals induced by human motor activity, are registered;

based on said signals from the three-component accelerometer, the magnitude of acceleration vector is calculated, time array of acceleration vector magnitudes is formed, and its extrema are identified;

duration of separate motor acts and the differences between adjacent extrema of acceleration vector magnitude for each motor act are determined by counting, consecutively from one extremum to another, the number of extrema equal to a reference number of extrema in at least one pattern, which is pre-formed for a certain type of motor activity and characterized by a specific reference number of acceleration vector magnitude extrema, a specific reference value of motor act duration, and specific reference values of difference between adjacent extrema of acceleration vector magnitude;

obtained values of motor act duration and difference between adjacent acceleration vector magnitude extrema are compared with reference values of motor act duration and difference between adjacent acceleration vector magnitude extrema from a corresponding pattern;

thereafter, the decision on the type of motor act performed is made if said determined values of motor act duration and difference between adjacent extrema of acceleration vector magnitude fit into preset ranges of said reference values of motor act duration and difference between adjacent extrema of acceleration vector magnitude of at least one pattern.

When analyzing the sequence of signals generated by a three-component accelerometer attached to the human body, the inventors have experimentally established that individual motor acts belonging to different types of motor activity are characterized by such informative parameters as:

number of acceleration vector magnitude extrema;

duration of motor act, and

values of difference between adjacent acceleration vector magnitude extrema.

Hence, it was proposed to form a number of patterns characterized by reference values of the number of acceleration vector magnitude extrema, motor act duration, and difference between adjacent acceleration vector magnitude extrema, and compare the corresponding parameters of acceleration vector magnitude registered in the process of implementing the proposed method therewith. Herein, natural deviation of such parameters as motor act duration and difference between adjacent acceleration vector magnitude extrema taken into account, the corresponding reference values are represented as a range of values - minimum and maximum. It should be noted that depending on the specific task, the number of patterns formed both for different and similar types of motor activity may vary, for example, a single pattern can be used if one type of motor activity has to be recognized.

While implementing the proposed method, the acceleration vector magnitude is calculated based on signals generated by a three-component accelerometer, then a time array of acceleration vector magnitude values is formed, and its extrema are identified.

Thereafter, the number of acceleration vector magnitude extrema equal to the reference number of extrema in the first pattern is counted starting from the first extremum, the duration of supposed motor act is measured, and the difference between adjacent extrema is determined. A similar procedure is consecutively performed from one acceleration vector magnitude extremum to another and repeated for each pattern. The values of motor act duration and difference between adjacent acceleration vector magnitude extrema, thus defined, are each time compared with corresponding reference values of a corresponding pattern.

The above procedure affords opportunity to consecutively analyze each fragment of the array of acceleration vector magnitude values by comparing its parameters with the reference ones, wherein the dimension of each analyzed fragment is determined by the reference number of extrema of the corresponding pattern.

Finally the decision on the type of motor act performed is made if said determined values of motor act duration and difference between adjacent extrema of acceleration vector magnitude characteristic for the analyzed fragment of acceleration vector magnitudes array fit into preset ranges of said reference values of motor act duration and difference between adjacent extrema of acceleration vector magnitude of the corresponding pattern.

The proposed motor act model, characterized by a number of acceleration vector magnitude extrema, motor act duration measured within this number of extrema, and difference between adjacent extrema and used in combination with the described procedures of isolating fragments of recorded time array of acceleration vector magnitudes and comparing said fragment parameters with the corresponding reference parameters (values), affords possibility to reliably identify the type of motor activity that the performed motor act belongs to and thus determine the type of motor activity in general. Here, the term "reliability" refers to the probability of errors of first and second kind, in other words, the probability of either false identification of the type of performed motor act, or identification uncertainty.

The reliability of identification of motor activity type can be enhanced, if variance of difference between adjacent acceleration vector magnitude extrema is taken into account with respect to corresponding reference values.

Given that human arms are involved in most types of motion, the accelerometer can be attached to person's arm, particularly to the wrist, which makes it possible to identify the majority of human motor activity types. Moreover, wearing such devices on the wrist is traditional and comfortable.

In a particular embodiment, the three-component accelerometer can be substituted with three interconnected acceleration sensors forming three orthogonal acceleration measurement axes.

High-frequency hopping of accelerometer signals characteristic for such application can be eliminated if signals generated by the three-component accelerometer or results of acceleration vector magnitude calculation are soothed before a time array of acceleration vector magnitudes is formed. Such a procedure further enhances the reliability of motor activity type identification and reduces the scope of computations necessary to implement said method.

Patterns, for example, can be formed as follows. Motor activity related signals from a three-component accelerometer attached to human body are recorded while the person performs a series of motor acts of a certain type, for which the pattern is formed. Further on, vector magnitudes are calculated based on said signals acceleration, their time array is formed, and its extrema are identified. Thereafter, the number of acceleration vector magnitude extrema, motor act duration and difference between adjacent extrema are determined for a selected number of motor acts. Finally, a repeating number of extrema, minimum and maximum duration of motor acts featuring the above number of extrema, and minimum and maximum difference between adjacent extrema in motor acts that have the above number of extrema, are taken as reference values for at least one pattern.

If motor acts with a different repeating number of extrema are identified, at least one additional pattern is formed, its reference values also being represented by the repeating number of extrema, minimum and maximum duration of motor acts featuring the above number of extrema, and minimum and maximum difference between adjacent extrema in motor acts characterized by the above number of extrema. The resulting patterns form a subset of patterns for one type of motor activity.

Additionally, to eliminate high-frequency hopping of signals generated by the three-component accelerometer said signals or calculated acceleration vector magnitudes are soothed before a time array of acceleration vector magnitudes is formed.

In general case, pattern formation can be disassociated from a particular person or type of motor activity to be determined by the proposed method. However, pattern forming yields the best results, if the three-component accelerometer is attached to the body of the same person and in the manner, which will be used when implementing the proposed method in future. In this case, patterns reflecting some peculiarities of certain person's motion can be formed.

In particular, the number of motor acts performed by a person during pattern formation ranges from 50 to 300.

Another object of this invention is a device used for determining types of motor activity and comprising a three-component accelerometer providing for its attachment on a human body and capable of generating signals that correspond to vector projections of the acceleration experienced by the accelerometer along three orthogonal space axes, and a computing device connected to the accelerometer outputs.

Said computing device is configured to perform all computations necessary to implement the proposed method. Namely:

accelerometer signal-based calculations of acceleration vector magnitude, formation of a time array of acceleration vector magnitudes, and identification of its extrema;

evaluation of individual motor act duration and difference between adjacent acceleration vector magnitudes for each individual motor act, with the boundaries of the motor acts being determined consecutively from one extremum to another by comparing the number of extrema with their reference number for at least one pattern pre-formed for a certain type of motor activity and characterized by a reference number of acceleration vector magnitude extrema, a reference duration of motor act, and reference values of difference between adjacent acceleration vector magnitude extrema;

comparison of measured motor act duration and difference between adjacent acceleration vector magnitude extrema with corresponding reference values of a certain pattern, and

making a decision on whether a motor act of certain type was preformed, provided that said measured values of motor act duration and difference between adjacent acceleration vector magnitude extrema fit into preset ranges of said reference values of motor act duration and difference between adjacent acceleration vector magnitude extrema of at least one pattern.

In a particular embodiment, said computing device may be configured to make decisions on whether a certain type motor act was performed by additionally taking into consideration the dispersion of difference between adjacent acceleration vector magnitude extrema with respect to corresponding reference values.

Said accelerometer can be designed to be fixed on the wrist.

Three structurally interconnected acceleration sensors forming three orthogonal acceleration measurement axes can be used instead of the accelerometer mentioned above.

In a particular embodiment, the computing device can be connected to accelerometer outputs via cyclic buffers to provide time correlation of signals at accelerometer outputs and their subsequent processing.

The device for determining types of human motor activity can be complemented with a transceiver to provide wireless transmission of data on the type of human motor activity being performed to an external device.

**BRIEF DESCRIPTION OF DRAWINGS**

The invention is illustrated by the following graphical materials.

Fig. 1 presents a block diagram of a sample device for implementing the proposed method for determining types of human motor activity in accordance with the present invention.

Fig. 2 shows an example for placing a device for determining types of human motor activity, a three-component accelerometer included, on a human wrist.

Fig. 3 presents a block diagram of a sample algorithm for implementing the method proposed by the claimed invention, wherein said algorithm is shown in general to illustrate basic operations of the method.

Fig. 4 shows a block diagram of a sample algorithm used to form patterns of various types of human motor activity.

Fig. 5 gives an example of graphical representation of temporal variations of acceleration vector magnitude, recorded by a three-component accelerometer. The example illustrates how local acceleration vector magnitude extrema that characterize various types of motor activity are determined.

Fig. 6 shows an enlarged view of fragment "c" of acceleration vector magnitude plot from Fig. 5 to illustrate the variation of acceleration vector magnitude within the boundaries of one motor act and the process of measuring the difference between adjacent extrema of acceleration vector magnitude.

Fig. 7 shows sample plots illustrating the dispersion of signals from a three-component accelerometer, which are recorded while implementing the proposed method.

Fig. 8A and Fig. 8B (continued) represent a block diagram of a sample algorithm for determining types of human motor activity that gives a more detailed description of the method, whose general block diagram is shown in Fig. 3.

Fig. 9 presents a block diagram of a sample algorithm for adjusting patterns of a particular type of human motor activity used for implementing the method according to the claimed invention.

## DETAILED DESCRIPTION OF THE PREFERRED EMBODIMENTS

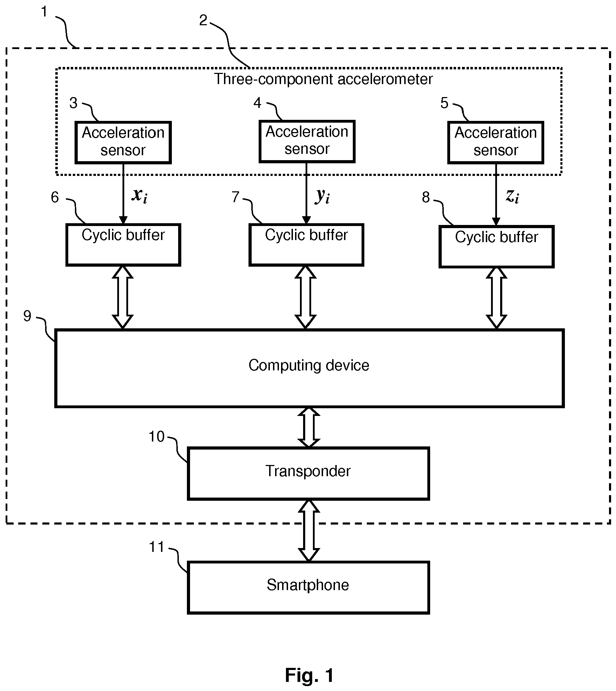

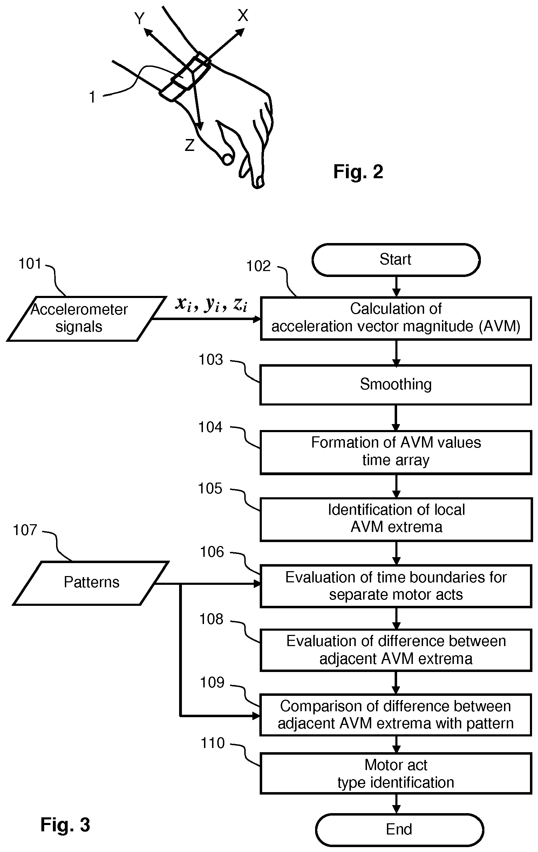

The method propsed by the claimed invention can be implemented, for example, by means of device 1, whose block diagram is shown in Fig. 1. Device 1 includes a three-component accelerometer 2 having three acceleration sensors 3, 4, 5 that generate signals corresponding to vector projections of the acceleration experienced by accelerometer 2 along three orthogonal axes in space $X$, $Y$ and $Z$. The outputs of acceleration sensors 3, 4 and 5 are connected to inputs of computing device 9 through corresponding cyclic buffers 6, 7 and 8 that accumulate the values of signals from acceleration sensors 3, 4 and 5 over a certain period of time. Device 1 can be attached to human body in such a way that human motor activity induces acceleration sensors 3, 4, 5 to generate steady signals. A human wrist is the most convenient place for attaching device 1, at least that part of the device that includes accelerometer 2, as shown in Fig. 2, thus affording opportunity to determine the largest number of different types of human motor activity, in other words, types of movement.

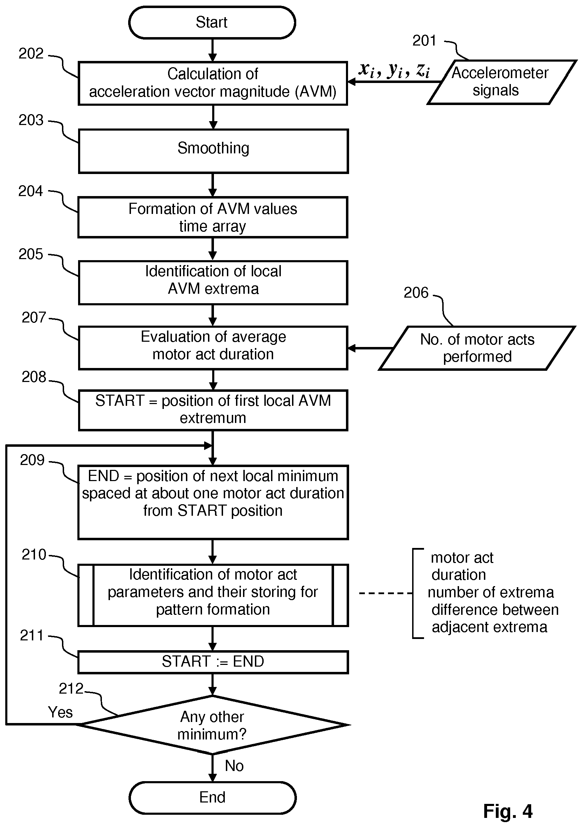

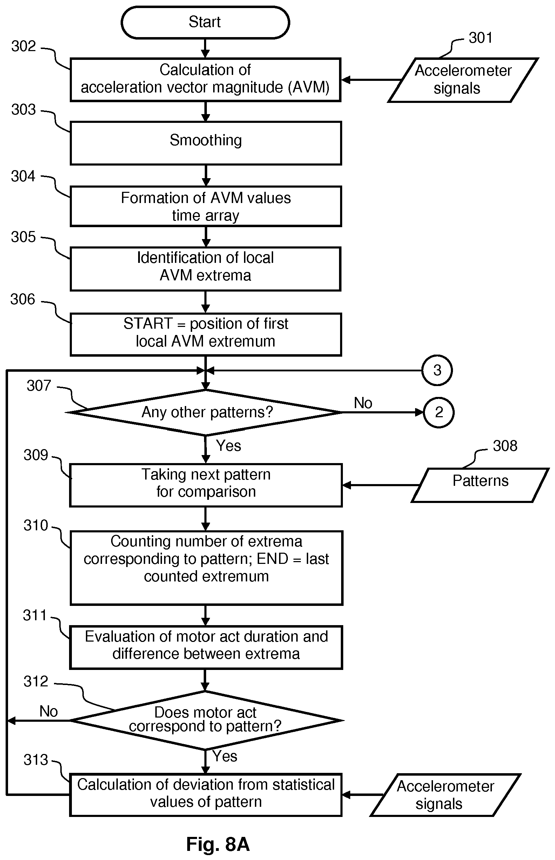

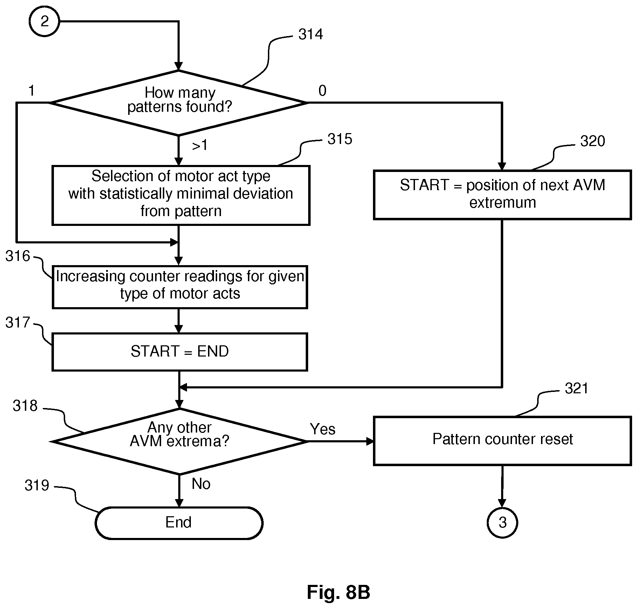

Three-component accelerometer 2 (see Fig. 1) continuously generates signals $x_i$, $y_i$ and $z_i$ (where $i$ is the consecutive count number) that correspond to acceleration projections experienced by accelerometer 2 along three orthogonal vector axes $X$, $Y$ and $Z$. Those signals are fed to computing device 9 through cyclic buffers 6, 7 and 8 for further processing. The cyclic (or circular) buffer is of FIFO (First In First Out) type commonly used to correlate data streams between some asynchronous processes, here - between continuously generated signals $x_i$, $y_i$, $z_i$ and their processing performed by computing device 9. Thus, cyclic buffers 6, 7, 8 store consecutive values of signals $x_i$, $y_i$, $z_i$, their total number being dependent upon the capacity of cyclic buffers 6, 7, 8; with said signal values continuously updated at each consecutive count. Computing device 9 determines particular types of motor activity based on the algorithms, whose block diagrams are presented in general in Fig. 4, and described in more detail in Fig. 8A, 8B. The capacity of cyclic buffers 6, 7, 8 is stipulated by a combination of such factors as processing speed of computing device 9, complexity of performed computations, and required accuracy of calculations.

Device 1 can be complemented with transceiver 10 for wireless transmission of data on currently performed human motor activity to an external stand-alone device, for example, to a personal computer or a smartphone 11, which accumulate those data. Other options are possible, in which data from smartphone 11 are transferred using certain network technologies, for example the Internet, to an external storage for accumulation and subsequent processing and analysis for the the above-mentioned purpose of assessing person's physical condition, bodily energy consumption, etc.

A block diagram, showing basic operations performed by the algorithm for implementing the proposed method is presented in Fig. 3. Signals $x_i$, $y_i$, $z_i$, where $i$ is the consecutive count number, are simultaneously generated at a given sampling rate and are fed to the outputs of three-component accelerometer 2 (position 101), which makes part of device 1 fixed on the wrist. As it was mentioned above, said signals correspond to projections of acceleration vector along three orthogonal space axes $X$, $Y$, $Z$.

At first (step 102), acceleration vector magnitude is calculated based on signals $x_i$, $y_i$, $z_i$ as:

$$A_i = \sqrt{x_i^2 + y_i^2 + z_i^2} \,,$$

thus forming a sequence of values $A_i$.

To eliminate high-frequency hopping of computed values $A_i$, the above sequence can be smoothed (step 103), for example, by method of exponential weighted moving average. Thereupon, a new array of values $\tilde{A}_i$ is formed, which is free of a high-frequency component. Various smoothing methods are known, which can be used depending on the frequency spectrum of signals $x_i$, $y_i$, $z_i$ generated by accelerometer 2.

Alternatively, the smoothing sequence and calculation of acceleration vector magnitude can be modified as follows: at first, signals $x_i$, $y_i$, $z_i$ are smoothed, after which the acceleration vector magnitude is computed.

Based on obtained values $\tilde{A}_i$, a time array of acceleration vector magnitudes is formed (step 104) and subsequently analyzed. Said time array is stored in the RW memory of computing device 9.

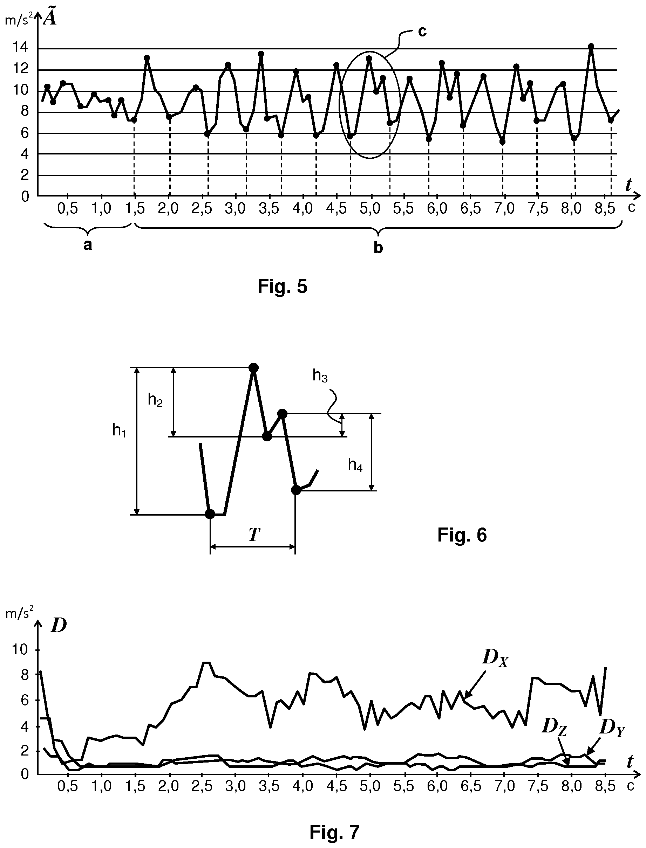

The values of acceleration vector magnitude $\tilde{A}_i$ are shown in Fig. 5 as a plot illustrating the changes of value $\tilde{A}_i$ in time. The curve shown features a characteristic serrated shape. In this example, amplitude and time ratios for $A_{\tilde{i}}$ in "a" region are characteristic for the case when a person, at least, does not walk or run, while those in "b" region are characteristic for a walking person.

In the next step 105 (see Fig. 3), local extrema of acceleration vector magnitude $\tilde{A}_i$ are determined (see dot marks on the plot of Fig. 5). Later, in step 106, time boundaries of individual motor acts are identified for the selected local extrema. Said identification is based on comparison of a series of local acceleration vector magnitude extrema $\tilde{A}_i$ with reference values of the number of acceleration vector magnitude extrema for one motor act within various types of human motor activity. The reference values are presented in Fig. 3, position 107.

A pattern, as noted above, presents a set of parameters or reference values characterizing a certain type of human motor activity. Thereat, a multitude of patterns related to a certain type of motor activity can be used, each of which, in its turn, can be represented by a subset of patterns for a given type of motor activity. For example, a multitude of patterns $S$ for recognizing walking, running, and jumping can include:

$$S = \{S_W, S_R, S_J\},$$

where: $S_W$ is a subset of walking patterns;

$S_R$ is a subset of running patterns;

$S_J$ is a subset of jumping patterns.

Besides, each subset $S_W$, $S_R$ or $S_J$ may include several patterns. For example, a subset of walking patterns $S_W$ comprising W patterns (where W is a natural integer) can be represented as:

$$S_W = \left\{S_W^{(1)}, S_W^{(2)}, ...., S_W^{(w)}\right\},$$

where: $S_W^{(1)}$ is the first walking pattern;

$S_W^{(2)}$ is the second walking pattern;

...

$S_W^{(w)}$ is the W-th walking pattern.

The number of local extrema per one motor act of a given type motor activity can be used as a criterion for distinguishing patterns of the same subset.

Finally, each pattern includes a set of the following reference values:

$$S = \{E, T_{\min}, T_{\max}, h_{(1)\min}, h_{(1)\max}, ..., h_{(E-1)\min}, h_{(E-1)\max}\},$$

where: $E$ is the number of extrema per one motor act (for example, a walking step, a running step, a jump, an arm swing, etc.);

$T_{\min}$ is the minimum duration of a motor act;

$T_{\max}$ is the maximum duration of a motor act;

$h_{(1)\min}$ is the minimum difference between the first and second extrema;

$h_{(1)\max}$ is the maximum difference between the first and second extrema;

$h_{(E-1)\min}$ is the minimum difference between the second-to-last and last extrema;

$h_{(E-1)max}$ is the maximum difference between the second-to-last and last extrema.

Those patterns are pre-formed at the stage of tuning or "teaching" device 1, which will be described below, and are stored as reference values in the memory of said computing device 9.

The time boundaries of individual motor acts are identified consecutively for all available patterns at step 106 (see Fig. 3), starting with the first detected local acceleration vector magnitude extremum. At first, extrema in quantity equal to the number of extrema $E$ in the first pattern are counted starting from the first local extremum, which is conditionally regarded as the beginning of the motor act. Thus, the duration $T$ of this conditional motor act is determined. If said duration $T$ does not fit within range $T_{min}$ - $T_{max}$ specified by the first pattern, the same procedure is performed for the next pattern. If duration $T$ of the first local extremum of acceleration vector magnitude matches no pattern with duration range $T_{min}$ - $T_{max}$, the same procedure is performed for the next local extremum acceleration vector magnitude. The same is repeated until a local minimum is found in the array of acceleration vector magnitude, which matches a relevant pattern, i.e., until duration $T$ per number of extremes $E$ in this pattern fits into the range of values $T_{min}$ - $T_{max}$ set by this pattern. As mentioned above, the number of patterns is determined by the complexity of the problem of recognizing various types of human motor activity, although, generally speaking, just a single pattern may be sufficient if a simple task to recognize a specific motor activity type by means of certain reference values is set.

Further on, within the time boundaries of motor act defined at step 106, the difference of adjacent extrema is determined (step 108), for example, from $h_1$ to $h_4$ for five extrema, as illustrated by the example in Fig. 6, which are later compared (step 109) with corresponding reference values of adjacent extrema differences $h_{(1)min}$, $h_{(1)max}$,..., $h_{(4)min}$, $h_{(4)max}$. The results of the comparison are stored in memory. The procedure is repeated for all patterns. If all compared differences of adjacent extrema of acceleration vector magnitude fit into a given range of reference values for a certain type of motor activity, a decision is made on whether a motor act corresponding to a given pattern or type of motor activity was performed.

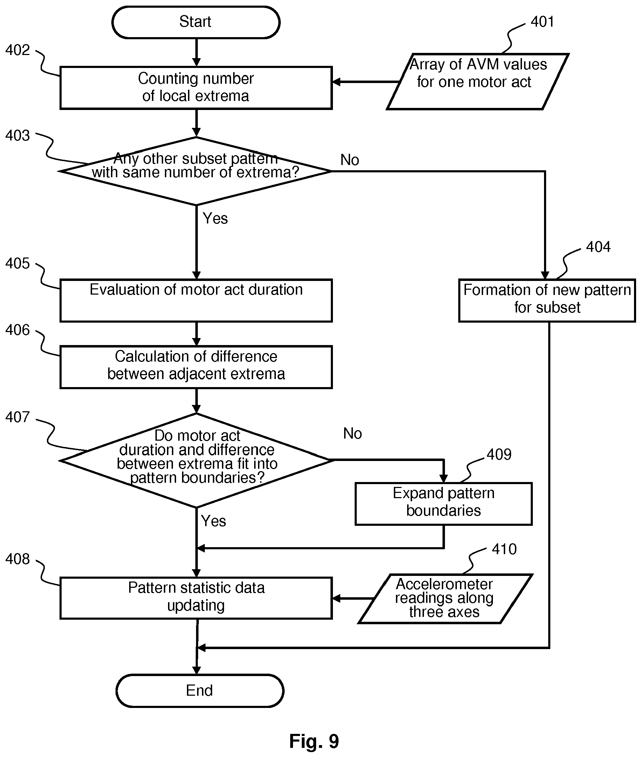

To form patterns, device 1 is attached to human body in the same manner as it will be used in future to determine motor activity type, for example, on the wrist, as shown in Fig. 2. The user switches device 1 to a setup mode and performs a specific type of motion, for example, walks or runs. A block diagram of a sample algorithm used for forming patterns for various types of human motor activity is shown in Fig. 4. The number of cyclic walking or running motor acts required to obtain reference values ranges from 50 to 300. If necessary, setting-up of device 1 can be repeated, for which a special mode for updating or adjusting reference values is provided; said mode will be described below in Fig. 9.

In pattern forming mode (see Fig. 4) and motor activity type identification mode, signals $x_i$, $y_i$, $z_i$ of accelerometer 2 (position 201) are used to calculate acceleration vector magnitude (step 202), whose values are then sequentially smoothed (step 203), as described above, and are used for forming a time array of acceleration vector magnitudes (step 204) and identifying local extrema of acceleration vector magnitudes (step 205).

Thereafter, the average duration of a given type motor act is measured (step 207) based on the number of performed motor acts of a given type (step 206). In particular, the plot from Fig. 5 drawn in the process of walking pattern formation, features dots that indicate acceleration vector magnitude extrema, and vertical dashed lines that show the temporal boundaries of a motor act, with local minima of acceleration vector magnitudes repeating at a roughly equal time intervals. The position of those local minima is considered the beginning of next motor act. As seen, one motor act - here, one walking step - lasts approximately 0.4 to 0.6 seconds.

Thereafter, the number of extrema per motor act is identified. As follows from Fig. 5, one motor act, selected for this example, can comprise three to five extrema, with the last extremum of the current motor act considered to be the first extremum of the next motor act. Thereafter, differences between adjacent extrema are specified. Said process is illustrated in Fig. 6, showing an enlarged view of fragment "c" of the acceleration vector magnitude plot presented in Fig. 5. Here, the number of extrema per motor act with duration $T$, is equal to five, while the differences between adjacent extrema are expressed as $h_1$, $h_2$, $h_3$, $h_4$, accordingly.

The above operations are performed in cycles: START (the beginning of cycle) corresponds to the position of a first local minimum (step 208), END indicates the position of the next local minimum spaced from the first local minimum (START position) by approximately the motor act duration (step 209). During the next step (210), the number of extrema within the motor act, motor act duration, and difference between the adjacent extrema within the motor act are determined. Those parameters are stored as initial reference values for the first pattern in a subset of walking patterns. Next, a checkout is performed (step 212) to see if other minima of acceleration vector magnitudes can be found within the approximate duration of a given type motor act. If another local minimum is detected, the cycle reverses to the beginning and the above mentioned process of specifying the number of extrema per given motor act, measuring the motor act duration and finding the difference between adjacent extrema within the motor act is repeated. Here, the beginning of cycle (START) corresponds to the position of the last local minimum related to the previous motor act.

The results are saved as next reference values. The process is repeated until no local minima in the time array of acceleration vector magnitudes is found at step 212.

A pattern subset for a given type of motor activity is finally formed as follows. The parameters stored at step 210 are sorted into groups having the same number of extrema, with the number of such groups being determined by the number of patterns in the subset. Minimum and maximum values of motor act duration $T_{min}$ and $T_{max}$ respectively, are determined for each group with the number of extrema equal to $E$, as well as minimum and maximum values of difference between adjacent extrema: $h_{(1)min}$, $h_{(1)max}$,..., $h_{(E-1)min}$, $h_{(E-1)max}$. Those parameters are accepted as reference. For example, for the case illustrated in Fig. 5, three patterns were formed depending on the number of extrema: 3, 4 and 5, identified in the array of acceleration magnitudes for individual motor acts.

A more detailed illustration of how the method proposed by the claimed invention can be implemented is given in Fig. 7 and Fig. 8a, 8b. Fig. 8a and 8b show a more detailed block diagram of the algorithm for determining types of human motor activity, which was generally described in Fig. 3. To enhance the reliability of determining types of motor activity, the algorithm additionally considers the dispersion of detected signals from the three-component accelerometer, for example, by calculating the mean value deviation magnitude.

As described above, at first, acceleration vector magnitude is calculated (step 302) based on accelerometer signals $x_i$, $y_i$, $z_i$ (step 301) and smoothed (step 303). Further on, a time array of acceleration vector magnitudes is formed (step 304) and local extrema of acceleration vector magnitudes are determined (step 305).

The process of determining typea of motor activity consists of cyclic comparisons of the time array of acceleration vector magnitudes with patterns. This process is performed in a manner described above and referenced to Fig. 3. It begins with identification of the first local extremum of acceleration vector magnitude (step 306). Thereafter, a checkout is performed (step 307) to see if other patterns are available that have not been compared with the time array of acceleration vector magnitudes. If such patterns are found (pattern data are denoted by position 308), the next pattern is taken for comparison (step 309), and a quantity of extrema from the array of acceleration vector magnitudes equal to the number of the given pattern extrema $E$ are counted out (step 310). Further on, duration $T$ and difference of adjacent extrema $h_1$, ..., $h_{E-1}$ are determined at step 311, after which a preliminary decision is made (step 312) as to whether a motor act and a given pattern are congruent by comparing the above values with reference values $T_{min}$ and $T_{max}$, as well as $h_{(1)min}$, $h_{(1)max}$,..., $h_{(E-1)min}$, $h_{(E-1)max}$. This decision is considered preliminary, since the analyzed fragment from the array of acceleration vector magnitudes may match two or more patterns. In fact, this stage only identifies tentative or candidate patterns, based on which a final decision on the type of motor act is made. Further on, pattern deviation from average statistical values is determined for this fragment (step 313) using current accelerometer signals. This information is stored in memory, while the procedure of selecting the next pattern, if such exists, is repeated alongside with procedures described in steps 309-312 and, if necessary, 313. If no correspondence is found, the pattern is excluded from the scope of candidates and the selection procedure is applied to a next pattern, if such exists, with the procedures specified in steps 309-312 and, if necessary, 313 being repeated.

As a result, one, two or no patterns are selected after all of them were examined. Thereafter, the final selection from revealed "candidates" is made or type of motor act is identified, optionally taking into account the deviation of difference between adjacent extrema from the average statistical values of the pattern.

An additional assessment of the deviation of those parameters from the statistical pattern values of may be made to enhance the reliability of motor activity type identification. An example of estimating the dispersion of accelerometer signals $x_i$, $y_i$, $z_i$ is given in Fig. 7 for a case of walking illustrated in Fig. 5. In this example, the dispersion, serving as a parameter for assessing the degree of deviation, is taken to be the average accelerometer signal deviation relative to the mean signal value. In this case, dispersion value $D_X$ of signal $x_i$ ranges from 2.5 to 9, and the average dispersion value $D_X$ is about 5. For signals $y_i$ and $z_i$ the value of dispersion $D_y$ and $D_z$ does not exceed 2, with the average being about 1. So, if average signal values $M(x_i)$, $M(y_i)$, $M(z_i)$ equal, respectively, to 6; 1.5; 1 are taken as reference values, then the deviation from the pattern will be:

$$D = (6-5)^2 + (1{,}5-1)^2 + (1-1)^2 = 1{,}25 .$$

The lower is value $D$, the more the performed motor act corresponds to the given pattern.

The final steps for determining the type of motor activity are described in Fig. 8b. At first (step 314), the number of candidate patterns that correspond to analyzed motor act are identified at step 312. If said number of candidate patterns is more than one, the analyzed motor act is considered to correspond to such type of motor activity that has a pattern, relative to which the value $D$ is minimal (step 315); thereafter, the count of this type of motor acts increases by one (step 316). If step 314 identifies only one candidate pattern, the process moves forward directly to step 316, increasing the count of this type motor acts by one. Step 317 marks the end of the cycle.

Further on, a checkout is performed to see if any other extrema can be found in the acceleration magnitude array (step 318). If none is detected, the process of determining the type of human motor activity is completed, and relevant counters store the information about the number of motor acts of a particular type identified. This information, taken as a whole, makes it possible to determine the type of human motor activity within a particular time interval.

If the results of step 314 show that no candidate pattern was identified at step 312, next extremum of acceleration magnitude is considered (step 320). Again, a checkout is performed to see if other extrema of acceleration magnitude are present (step 318). If none is detected, the identification of motor activity type is completed. If other extrema are found in acceleration magnitude array, the pattern counter is reset (step 321) and step 307 is undertaken again (see Fig. 8a).

If necessary, the user can switch to pattern updating mode. This may be required, for example, if device 1 is attached to the other arm or a different place on user's body, or when it changes users. Updating of patterns may be required to identify motor activity types when the user moves is a specific manner.

A block diagram of the operation algorithm of device 1 in pattern updating mode is shown in Fig. 9. At first, a pattern, or rather a pattern subset to be updated is specified. For example, a walking pattern subset needs to be updated. The user switches device 1 to an update mode and performs from 50 to 300 motor acts of certain type (here - walking steps), same as during pattern forming procedure. Thus, an array of acceleration vector magnitudes is formed, as described above with references to Fig. 3, 4, 8a, 8b. Further on, updating of subset patterns is described by an example of processing a fragment of acceleration vector magnitude array for a separate motor act (position 401).

Initially, the number of extrema in said fragment of acceleration vector magnitude array is identified (step 402). Thereafter, a checkout is performed (step 403) to see if any other previously formed patterns of the subset have the same number of extrema. If none is detected, a new pattern is formed (step 404) for a given pattern subset. In this case, the reference values include: for reference number of extrema: a new number of extrema; for reference minimum and maximum motor act duration: fragment duration; for minimum and maximum difference between adjacent extrema: corresponding differences between adjacent extrema of a given fragment of acceleration vector magnitude array. This marks the completion of new pattern formation. Its reference values regarding motor act duration range and the range of difference between adjacent extrema will be adjusted in future.

If step 403 establishes that among previously formed patterns of a given subset there exists a pattern with a number of extrema $E$ equal to that found in analyzed fragment of acceleration vector magnitude array, its reference values need updating. At first, the fragment duration, i.e., the duration of motor act (step 405) is evaluated, and then (step 406) the difference between adjacent extrema of acceleration vector magnitudes is calculated. Further on, a checkout is performed (step 407) to see if the calculated motor act duration and difference between adjacent extrema fit into the ranges of reference values: $T_{min}$, $T_{max}$, and also $h_{(1)min}$, $h_{(1)max}$,..., $h_{(E-1)min}$, $h_{(E-1)max}$. If so, the statistical data of the pattern are updated:at at step 408. $M(x_i)$, $M(y_i)$, $M(z_i)$ . If at least one of the obtained values of motor act duration and difference between adjacent extrema does not fit into the range of corresponding reference value, step 409 is performed to expand said range of values by changing the corresponding value from $T_{min}$, $T_{max}$, or $h_{(1)min}$, $h_{(1)max}$,..., $h_{(E-1)min}$, $h_{(E-1)max}$, after which the statistical data $M(x_i)$, $M(y_i)$, $M(z_i)$ of the pattern are updated. Accelerometer signals (step 410) are used to update the statistical data of the pattern. This marks the end of pattern updating.

The method for determining types of human motor activity in accordance with the claimed invention makes it possible to identify not only walking or running as described in examples above, but other types of movement too, for example, jumping or motionless resting. Identification of such types of motor activity as "vertical arm swing" or "horizontal arm swing" can be used, among other, to determine the type of physical exercise or actions being performed by a person. The proposed method can be used in a variety of applications. Checking the condition of user's body, for example, by monitoring user's motor activity seems the most promising application for this method.

The described device intended for implementing the proposed method can be manufactured as a wristband that identifies types of movement, counts the number of motor acts performed and monitors user's health. The invention can be embodied as a separate gadget or as a system comprising a man-wearable device equipped with acceleration sensors and acceleration signal processing circuit, mobile communication means, and an external database.

**Claims**

1. A method for determining types of human motor activity comprising:

   registering signals from a three-component accelerometer attached to human body and capable of generating signals induced by human motor activity;
   calculating a magnitude of an acceleration vector based on said signals from the three-component accelerometer, forming a time array of acceleration vector magnitudes and identifying extrema of the time array;
   determining durations of separate motor acts and differences between adjacent extrema of an acceleration vector magnitude for each motor act by consecutively counting from one extremum to another a number of extrema equal to a reference number of extrema in at least one pattern, the at least one pattern being predetermined for a certain type of motor activity and **characterized by** a specific reference number of an acceleration vector magnitude extrema, determining a specific reference value of motor act duration, and determining specific reference values of a difference between adjacent extrema of the acceleration vector magnitude;

comparing determined values of durations of motor acts and the difference between adjacent acceleration vector magnitude extrema with reference values of motor act duration and the difference between adjacent acceleration vector magnitude extrema of a corresponding pattern; and

determining a type of a motor act performed depending on whether said determined values of the durations of the motor acts and the difference between the adjacent extrema of the acceleration vector magnitude fit into preset ranges of said reference values of the motor act duration and said differences between the adjacent extrema of acceleration vector magnitude of at least one pattern.

2. The method of claim 1, further comprising determining that a motor act of a known type was performed depending on dispersion of the differences between the adjacent extrema of acceleration vector magnitude with respect to corresponding pattern values.

3. The method of claim 1, wherein said three-component accelerometer is attached to a human arm.

4. The method of claim 3, wherein said three-component accelerometer is fixed on a wrist.

5. The method of claim 1, wherein the three component accelerometer comprises three structurally interconnected acceleration sensors forming three orthogonal acceleration measurement axes.

6. The method of claim 1, wherein signals from the three-component accelerometer or the acceleration vector magnitudes are subjected to a smoothing procedure prior to the formation of the time array of the acceleration vector magnitudes.

7. The method of claim 1, wherein forming the at least one pattern comprises:

recording signals from the three-component accelerometer attached to a body of a human and capable of generating signals induced by human motor activity; said recording being performed during a series of human motor acts of the type for which the at least one pattern is formed;

calculating a magnitude of an acceleration vector based on said signals from the three-component accelerometer, forming a time array of acceleration vector magnitudes and identifying extrema of the time array;

for each motor act determining a number the extrema of the acceleration vector magnitude, a duration of each motor act and a difference between adjacent extrema of the acceleration vector magnitude; and

designating as reference values of the at least one pattern a recurring number of the extrema, minimum and maximum values of the duration of each motor act having the recurring number of the extrema, and minimum and maximum values of the difference between the adjacent extrema having the recurring number of extrema.

8. The method of claim 7, wherein at least one additional pattern is formed when at least one motor act is found to contain a different recurring number of extrema, wherein the different recurring number of extrema, minimum and maximum values of a motor act duration having the different recurring number of extrema, and minimum and maximum values of a difference between adjacent motor act extrema having the different recurring number of extrema are taken as reference values of the at least one additional pattern.

9. The method of claim 7, wherein signals from the three-component accelerometer or the acceleration vector magnitudes are subjected to a smoothing procedure prior to the formation of the time array of the acceleration vector magnitudes.

10. The method of claim 7, wherein forming the at least one pattern further comprising attaching the three-component accelerometer to the body of the human whose types of human motor activity are then determined.

11. The method of claim 7, wherein the number of motor acts in a series of the same types of the motor acts performed by the human during forming the at least one pattern ranges from 50 to 300.

12. A device for determining types of motor activity comprising:

a three-component accelerometer for attaching to a body of a human and for generating signals corresponding to projections of an acceleration vector of the accelerometer along three orthogonal space axes, and a computing device coupled to outputs of the accelerometer;

the computing device serving to compute an acceleration vector magnitude, to form a time array of acceleration vector magnitudes, and to identify its extrema;
determining duration of individual motor acts and determining differences between adjacent extrema of the acceleration vector magnitude for each individual motor act, wherein the boundaries of the motor acts are determined consecutively from one extremum to another by comparing the number of extrema with their reference number for at least one pattern pre-formed for a known type of motor activity and **characterized by** a reference number of acceleration vector magnitude extrema, a reference duration of a motor act, and reference values of a difference between adjacent extrema of the acceleration vector magnitude;
comparing of measured durations of the motor act and the difference between the adjacent extrema of the acceleration vector magnitude with corresponding reference values of a corresponding pattern; and
determining whether a given type of a motor act was preformed, provided that said measured values of motor act duration and difference between the adjacent extrema of the acceleration vector magnitude fit into preset ranges of said reference values of durations of the motor act and the difference between the adjacent extrema of the acceleration vector magnitude of the at least one pattern.

13. The device of claim 12, wherein the computing device is configured to determine the type of motor act performed with consideration for dispersion of differences between adjacent extrema of acceleration vector magnitude relative to corresponding reference values.

14. The device of claim 12, wherein said accelerometer can be attached on the wrist.

15. The device of claim 12, wherein the three component accelerometer comprises three structurally interconnected acceleration sensors forming three orthogonal acceleration measurement axes.

16. The device of claim 12, wherein the computing device is coupled to accelerometer outputs via cyclic buffers.

17. The device of claim 12, further comprising a transceiver to provide wireless transmission of data on currently performed type of human motor activity to an external device.

**Fig. 1**

**Fig. 2**

```
Start
  │
  ▼
```

101 — Accelerometer signals

$x_i, y_i, z_i$

102 — Calculation of acceleration vector magnitude (AVM)

103 — Smoothing

104 — Formation of AVM values time array

105 — Identification of local AVM extrema

107 — Patterns

106 — Evaluation of time boundaries for separate motor acts

108 — Evaluation of difference between adjacent AVM extrema

109 — Comparison of difference between adjacent AVM extrema with pattern

110 — Motor act type identification

End

**Fig. 3**

Fig. 4

Fig. 5

Fig. 6

Fig. 7

**Fig. 8A**

**Fig. 8B**

Fig. 9

## INTERNATIONAL SEARCH REPORT

| International application No. |
| --- |
| PCT/RU 2016/000098 |

### A. CLASSIFICATION OF SUBJECT MATTER

A61B 5/11 (2006.01)

According to International Patent Classification (IPC) or to both national classification and IPC

### B. FIELDS SEARCHED

Minimum documentation searched (classification system followed by classification symbols)

A61B 5/11, 5/103

Documentation searched other than minimum documentation to the extent that such documents are included in the fields searched

Electronic data base consulted during the international search (name of data base and, where practicable, search terms used)

"Rossiiskaya meditsina", CIPO, DEPATISnet, DWPI, EAPATIS, Espace, Espacenet, Google, KIPRIS, Medline, PAJ,

### C. DOCUMENTS CONSIDERED TO BE RELEVANT

| Category* | Citation of document, with indication, where appropriate, of the relevant passages | Relevant to claim No. |
| --- | --- | --- |
| D, A | US 2011/0098583 A1 (TEXAS INSTRUMENTS INCORPORATED) 28.04.2011, fig. 2, 3, 8, 13-14, 24, para. [0153] - [0154], [0157], [0176], [0189]-[0190], [0200], [0250] - [0251], fig. 40 A, B | 1-17 |
| A | JP 2012065749 A (CITIZEN HOLDINGS CO LTD et al.) 05.04.2012, the abstract, fig. 6-9 | 1-17 |

☐ Further documents are listed in the continuation of Box C.     ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 02 June 2016 (02.06.2016) | 30 June 2016 (30.06.2016) |

| Name and mailing address of the ISA/ RU | Authorized officer |
| --- | --- |
| Facsimile No. | Telephone No. |

Form PCT/ISA/210 (second sheet) (July 1998)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013143996 B
- US 20130245470 A

- JP 2012065749 B